# EUROPEAN PATENT APPLICATION

(11) **EP 4 814 488 A1**
(43) Date of publication of application: **30.09.2026**
(21) Application number: 25315109.6
(22) Date of filing: 28.03.2025
(51) Int. Cl.: G01N 33/50

(54) **USE OF GOVS TO EVALUATE ORGANELLE DOMAIN MODULATORS**

(71) Applicant: Paris Sciences et Lettres, 75006 Paris (FR); Centre National de la Recherche Scientifique, 75016 Paris (FR); Université Paris Cité, 75006 Paris (FR); Sorbonne Université, 75006 Paris (FR)
(72) Inventor: Thiam, Abdou Rachid, 75005 Paris (FR); Zouiouich, Mehdi, 75005 Paris (FR); Omrane, Mohyeddine, 75005 Paris (FR); Bhapkar, Apoorva, 75014 Paris (FR)
(74) Representative: Cabinet Chaillot

(57) **Abstract**

The present invention concerns a new method for screening the activity of a molecule involved in regulating organelle domains, as well as a new method for modulating organelle domain formation. It further concerns the use of the giant organelle vesicles for studying organelle domain formation or in a method for screening the activity of a molecule or combination of molecules involved in the modulation of organelle domains. The invention also concerns a screening platform for identifying domain-modulating molecules in organelles and the molecules or combination of molecules identified by the screening method of the invention for use in the prevention and/or the treatment of diseases, disorders, or deficiencies related to the protein regulation and/or lipid metabolism.

## Description

### Background of the invention

The invention concerns a new use of giant organelle vesicles (GOVs) in applications including methods of screening the activity of molecules involved in the regulation of organelle domains and methods for modulating organelle domain formation. The invention also concerns the use of giant organelle vesicles to identify a molecule or combination of molecules involved in the modulation of organelle domains, for instance molecules involved in the regulation of the INSIG/SCAP/SREBP complex in the endoplasmic reticulum.

The inventors have previously developed giant organelle vesicles (GOVs) derived from native intracellular organelles such as lysosomes, endosomes, mitochondria, Golgi and endoplasmic reticulum (ER).

EP4166131 describes the recovery method of stable and functional giant organelles from cells and the giant organelles obtained therefrom. It describes the method of screening the activity of a molecule of interest, especially its capacity to interact with a least one protein on a giant organelle, to identify molecules targeting giant organelles, and to test the toxicity of molecules blocking the activity of giant organelles.

US 18/273,728 describes an automated method for identifying a compound that impacts a characteristic of a lipid raft phase domain, a characteristic of a non-raft phase domain and/or a characteristic of one or more membrane proteins using a population of vesicles. The vesicles can be a Giant Plasma-Membrane Derived Vesicle (GPMV).

None of these documents describes the present applications with GOVs derived from intracellular organelles such as lysosomes, endosomes, mitochondria, Golgi and endoplasmic reticulum. The inventors wish to emphasize that the GPMVs developed the method of EP4166131 is different from GPMVs in US 18/273,728, using classical GPMVs' method of production.

These particular GOVs of the invention represent the perfect system to study domain formation as a proxy for cellular state and function. They can be used to assess the self-assembly of lipids and proteins which is important for catalysing cooperative cellular reactions. Domains can be read by the usage of fluorescent reporters such as Laurdan or DiI (1,1'-dioctadecyl-3,3,3',3'-tetramethylindocarbocyanine).

Since domains regulate specific reactions whose impairment leads to diseases, being able to probe domain formation in organelles can allow the development of strategies to fight against a variety of diseases.

Moreover, since GOVs can be generated in large quantities, with or without the addition of certain types of exogenous molecules, they represent a powerful low-cost tool to study lipid domains and the modulation of their components.

### Description of the invention

Taking advantage of the GOVs system, the inventors have developed an approach using giant ER vesicles (GERVs) where the phase separation of lipids and specific proteins into localized domains indicates the retention of the key enzyme SCAP/SREBP which is essential for cholesterol synthesis and uptake. The plasma membrane, endosomes, lysosomes (GPMV, GEVs and GLVs) also display domains like GERVs.

We present here GERVs as a study case.

By supplementing GERVs with various molecules, such as lipids or inhibitors, the inventors can identify conditions which promote or inhibit the sequestration of SCAP/SREBP. This tool provides a straightforward test to identify modulators of domain formation and to manipulate protein spatial localization within domains for pharmaceutical purposes.

Lipid domains have been studied for many years, mostly on the plasma membrane. It has been shown that these domains are important for various cellular processes as they can control the localization and activation of some proteins. However, the formation and modulation of domains on other organelle membranes, such as the ER, are not well understood. Whenever domains have previously been observed on ER-derived organelles, they required cooling down below room temperature and/or using protein-derived probes (King et al., PNAS 2020). However, the relevance of these domains at physiological temperature was not shown and the vesicles were strictly inside the cells. Moreover, the authors did not try to modulate the domains either, by adding drugs or molecules.

SREBP proteins are crucial in cell metabolism and growth and are dysregulated in many diseases. Due to the limitations of the current systems, there is little understanding of how SCAP/SREBP and eventually other ER-resident enzymes are spatially controlled. Targeting the activity of these proteins may help reduce the burden of frequent pathologies such as obesity or atherosclerosis or improve the outcome of patients with certain types of cancer.

Here the inventors provide a way to generate giant organelle vesicles (GOVs) that can be used to observe and modulate domains. They developed the technique on ER vesicles (GERVs) and by adding lipids or drugs, they were able to regulate the formation of domains at physiological temperatures. They were able to reduce SREBP cleavage, and as a result, reduce lipid synthesis. They have deducted that SCAP binds to cholesterol and 25-hydroxycholesterol (25HC) which favors the binding of INSIG proteins to SCAP, retaining the SCAP-SREBP complex in the ER. The interaction of INSIG/SCAP/SREBP complex with 25HC and cholesterol was previously known (Matsuda et al., Genes Dev 2001, Chandrasekaran et al., Int. J. Mol. Sci. 2024), but what the inventors were able to show is that without cholesterol ester-rich domains, these interactions do not seem to be enough to sequester SREBP in the ER.

Compared to existing technologies, the present method provides a physiologically relevant environment to study and manipulate GOV domains, e.g., GERV domains, offering more insights and potential therapeutic perspectives.

### Detailed description of the invention

The present invention relates to a method for screening the activity of a molecule involved in the regulation of organelle domains, said method comprising the following steps:
a) providing giant organelle vesicles, GOVs ;
b) supplementing the GOVs with at least a molecule to be tested;
c) screening the molecule based on its capacity to induce a modification of the organelle domains in the GOVs after the supplementation,
wherein the giant organelle vesicles are chosen from the group consisting of the endoplasmic reticulum, mitochondria, lysosome, nucleus, Golgi apparatus, vacuole, chloroplast, autophagosome, autolysosomes, endosomes, plasma membranes, peroxisome, multivesicular bodies.

The inventors have developed a method based on osmotic swelling to generate intracellular organelles having an increased size compared to their original form, otherwise called giant organelles or giant organelle vesicles, then generating a plasma membrane tension increase of swollen cells leading to lysis of the plasma membrane and the release of the cell interior, in particular stable and functional giant organelles vesicles. This technique is described in detail in EP patent application EP 21202428.5 and PCT application WO/2023/062149.

The molecule to be tested can be added before the osmotic swelling of the cells, therefore within the live cells medium, or after the osmotic swelling of the cells and organelles into an hypotonic medium.

As such, in an alternative embodiment, the supplementation step b) with at least a molecule to be tested is performed in the live cells medium, before the formation of the GOVs of step a) following the osmotic swelling of the cells and organelles into an hypotonic medium.

The supplementation with the molecule to be tested can therefore be performed on cells, before the osmotic swelling, or on the GOVs newly formed.

The screening step c) can be performed using fluorescence approaches revealing changes in membrane properties, e.g., via plate readers or fluorescence microscopy. It can also be performed using non-flurorescent methods based for instance on phase constrast, Raman, or diffraction properties of GOVs.

The giant organelle vesicles are obtained with the method disclosed in EP 21202428.5 and PCT application WO/2023/062149 where the term "giant organelle vesicles" (GOVs) means organelles having a mean size generally over 1 µm, from about 1 to 50 µm, preferably from about 2 to 15 µm, more preferably from about 3 to 10 µm, the most preferably of about 5 µm. Such giant organelles have a surface-to-volume ratio decreased compared to their native form which ranges from about 3 µm¹ to about 0,06 µm⁻¹, preferably from about 1,5 µm⁻¹ to about 0,15 µm⁻¹, more preferably from about 0,3 µm⁻¹ to about 1 µm⁻¹. Such giant organelles have a membrane tension above 10⁻⁶mN/m and below 10mN/m.

By "modification of the organelle domains" is meant that the formation or the dissolution of domains or even the modification of the domains' components can be induced in response to the exposure to the test molecule.

The giant organelle vesicles can effectively be generated from the endoplasmic reticulum, mitochondria, lysosome, nucleus, Golgi apparatus, vacuole, chloroplast, the autophagosome, autolysosomes, endosomes, plasma membranes, peroxisome, multivesicular bodies.

According to a particular embodiment of the method of the present invention, the giant organelle vesicles can be giant endoplasmic reticulum vesicles (GERVs). As a result, the method for screening the activity of a molecule, wherein the giant organelle vesicles are giant endoplasmic reticulum vesicles (GERVs) comprises the following steps :
a) providing giant endoplasmic reticulum vesicles (GERVs) ;
b) supplementing the GERVs with at least a molecule to be tested;
c) screening the molecule based on the modification of the ER domains in the GERVs after the supplementation.

The molecule to be tested can be added before the osmotic swelling of the cells, therefore within the live cells medium, or after the osmotic swelling of the cells and organelles into an hypotonic medium.

Therefore, in an alternative embodiment, the supplementation step b) with at least a molecule to be tested is performed in the live cells medium, before the formation of the GERVs of step a) following the osmotic swelling of the cells and organelles into an hypotonic medium.

According to a particular embodiment of the method of the present invention, the molecules to be tested can be chosen from the group consisting of molecules involved in protein regulation and lipid metabolism.

For instance, the molecules to be tested can be, but not limited to, hypolipidemic agents, anticancer agents, lipid-based molecules, polyunsaturated lipids, saturated lipids, membrane lipids, SPT (serine palmitoyltransferase) inhibitors, ACAT (acyl-CoA cholesterol acyltransferase) inhibitors, NCEH1 (Neutral cholesterol ester hydrolase 1) inhibitors, lipid synthesis enzyme inhibitors or stimulators.

According to a particular embodiment of the method of the present invention, the molecules to be tested are chosen from the group consisting of molecules involved in the metabolism of cholesterol such as molecules involved in the reactions of esterification of cholesterol, hydrolysis of cholesterol ester back to free cholesterol and fatty acids, and their regulation, molecules involved in the metabolism of saturated lipids or molecules involved in the metabolism of sphingolipids.

Such molecules can be, but not limited to, those which are used in research on cholesterol metabolism such as U18666A (Sigma-Aldrich, 662015), a cholesterol transport inhibitor, Sandoz 58-035 (Sigma-Aldrich, S9318), an inhibitor of acyl-CoA cholesterol acyltransferase.

The present invention also relates to a method for modulating organelle domain formation, said method comprising the following steps :
a) providing giant organelle vesicles, GOVs ;
b) supplementing the GOVs with at least a molecule identified in the previously described screening method as having an effect on the domain formation, composition, or properties ;

wherein said domains are to be used for studying cellular processes dependent on the localization and activation of specific proteins,
wherein the giant organelle vesicles are chosen from the group consisting of the endoplasmic reticulum, mitochondria, lysosome, nucleus, Golgi apparatus, vacuole, chloroplast, the autophagosome, autolysosomes, endosomes, plasma membranes, peroxisome, multivesicular bodies.

The molecule to be tested can be added before the osmotic swelling of the cells, therefore within the live cells medium, or after the osmotic swelling of the cells and organelles into an hypotonic medium.

As such, in an alternative embodiment, the supplementation step b) with at least a molecule to be tested is performed in the live cells medium, before the formation of the GOVs of step a) following the osmotic swelling of the cells and organelles into an hypotonic medium.

By "modulating organelle domain formation" is meant that the formation of organelle domains can either be inhibited or activated under the influence of the supplied molecule. The inhibiting molecules will down regulate the formation of domains, whereas the activating molecules will up regulate the formation of domains of interest. Molecules can also promote domains but change their composition and properties, leading to a change in the domain protein content.

In a particular embodiment, the invention relates to a method for modulating organelle domain formation, said method comprising the following steps:
a) providing giant endoplasmic reticulum vesicles, GERVs, in particular ;
b) supplementing the GERVs with at least a molecule identified in any one of the methods of screening of the invention as having an effect on the domain formation, composition, and properties,
wherein said domains are to be used for studying cellular processes dependent on the localization and activation of specific proteins.

The present invention also relates to a use of giant organelle vesicles for studying organelle domain formation, wherein the giant organelle vesicles are chosen from the group consisting of the endoplasmic reticulum, mitochondria, lysosome, nucleus, Golgi apparatus, vacuole, chloroplast, the autophagosome, autolysosomes, endosomes, plasma membranes, peroxisome, multivesicular bodies.

The present invention also relates to a use of giant organelle vesicles in a method for screening the activity of a molecule or combination of molecules involved in the modulation of organelle domains.

According to a particular embodiment of the use of the present invention, the giant organelle vesicles can be endoplasmic reticulum vesicules (GERVs) and the molecule or combination of molecules is involved in modulating ER domains.

According to a particular embodiment of the use of the present invention, the molecule or combination of molecules is involved in the regulation of the INSIG/SCAP/SREBP complex in the endoplasmic reticulum.

As discussed above SREBP proteins are crucial in cell metabolism and growth and are dysregulated in many diseases. SREBP proteins are involved in de novo cholesterol and fatty acid synthesis. Several diseases, such as atherosclerosis, MASLD, type 2 diabetes, neurodegenerative diseases and cancer like hepatocellular carcinoma are known to be related to SREBP activity (Shimano et al., Nat. Rev. Endocrinol., 2017, Brown et al., Cell, 1997).

According to a particular embodiment of the use of the present invention, the molecules can be chosen from the group comprising a combination of cholesterol and acyl-CoA, an inhibitor of ACAT, such as Sandoz 58-035, an inhibitor of NCEH1, an inhibitor of SPT, an inhibitor of DGAT, lipid synthesis enzyme inhibitors.

The present invention also relates to a screening platform for identifying domain-modulating molecules in organelles, wherein said platform uses the giant organelle vesicles technology and wherein the giant organelle vesicles are chosen from the group consisting of the endoplasmic reticulum, mitochondria, lysosome, nucleus, Golgi apparatus, vacuole, chloroplast, the autophagosome, autolysosomes, endosomes, plasma membranes, peroxisome, multivesicular bodies.

Such screening platforms can use, but are not limited to, high-throughput screening HTS technology, microfluidics screening technology, bulk screening technology, and all microscopy-based screening technology suitable for the screening method of the present invention.

The present invention also relates to a molecule or combination of molecules identified by the screening method of the present invention for use in the prevention and/or treatment of diseases, disorders, or deficiencies related to the protein regulation and/or lipid metabolism.

According to a particular embodiment of the present invention, the molecule or combination of molecules is for use in the prevention and/or treatment of diseases, disorders, or deficiencies related to cholesterol metabolism.

Such diseases, disorders or deficiencies can be any one of the previously cited ones, such as cancer, metabolic disorders, neurodegenerative disorders, MASLD, type 2 diabetes, but not limited thereto.

To better illustrate the subject of the present invention, a description is given below of non-limiting, illustrative examples in connection with the appended figures.

### Brief description of the figures

Figure 1 represents bright-field (left panel) and fluorescence confocal microscopy imaging (remaining three panels) of an isolated GERV with an ER marker in magenta (KDEL-RFP) and eGFP-SCAP in green in absence of feeding (upper panels) and when fed with cholesterol (lower panels). Scale bar = 3 µm.
Figure 2 represents fluorescence confocal microscopy imaging of an isolated GERV with eGFP-SCAP with DMSO as a control (1^{st} panel), with the addition of acyl-CoA (2^{nd} panel), with the addition of cholesterol (3^{rd} panel) and with the addition of both acyl-CoA and cholesterol (4^{th} panel).
Figure 3 represents fluorescence confocal microscopy imaging of isolated GERVs with an ER marker in magenta (KDEL-RFP) and eGFP-SCAP in green, pre- and post-addition of cholesterol + an acyl-CoA (oleoyl-CoA). The sequential images are shown from top to bottom: merger to show the localization of both markers, pre-addition, post-addition, 150 seconds after addition and 250 seconds after addition.

The broken lines in the bottom images embody the formation of the domains.

The left panel is without inhibition, the middle panel with an ACAT inhibition and the right panel is with DGAT inhibition.

### Examples

### 1) ER domains formation on GERVs

### Materials and methods

The method of recovery of GOVs is described in European patent application EP4166131 and in PCT application WO/2023/06149.

The method is adapted for the recovery of GERVs as follows: Huh7 cells were plated on MatTek petri dishes (MatTek, P35G-1.5-14-C) and allowed to grow for 24h. Cells were then co-transfected with RFP-KDEL and GFP-SCAP using X-tremeGENE HP DNA (Roche, XTGHP-RO) . 24h after transfection, cholesterol was added to one condition for 2h at 200 µM. GERVs were obtained by using the same process as described in the above-mentioned patent applications. Images were acquired on a Zeiss LSM 880 confocal microscope (63x, NA 1.4) .

Figure 1 shows isolated GERVs with an ER marker in magenta (RFP KDEL) and GFP-SCAP in green. This figure shows the formation of the domains in a cholesterol-dependent manner. Indeed, by adding cholesterol to the cells, the SCAP signal becomes heterogeneous around the vesicle, which shows that SCAP is found in ER domains.

### 2) ER domain modulation using exogenous molecules on GERVs Materials and methods

Huh7 cells were plated on MatTek petri dishes (MatTek, P35G-1.5-14-C) and allowed to grow for 24h. Cells were then transfected with GFP-SCAP using X-tremeGENE HP DNA (Roche, XTGHP-RO). 24h after transfection, the ACAT inhibitor Sandoz 58-035 was added to the media of the cells at a final concentration of 2 µg/mL for 2 hours before swelling. GERVs were obtained by using the same process as described above. Sandoz 58-035 was added back to the hypotonic media at a final concentration of 2 µg/mL. DMSO, acyl-CoA (oleoyl-CoA), cholesterol, or mix of both lipids were added to the hypotonic media containing the GERVs. GERVs were incubated at 37°C for 20min before imaging. Images were acquired on a Zeiss LSM 880 confocal microscope (63x, NA 1.4).

Figure 2 shows isolated GERVs with GFP-SCAP in green. This figure shows the formation of the domains only after the addition of cholesterol + acyl-CoA to stimulate cholesterol ester (CE) synthesis.

The first 3 panels show that the formation of ER domains is not observed by adding either DMSO, acyl-CoA or cholesterol alone. By adding a mix of acyl-CoA + cholesterol to the GERVs, the SCAP signal becomes heterogeneous around the vesicle, which shows that SCAP is found in ER domains.

Figure 3 shows isolated GERVs with an ER marker in magenta (RFP-KDEL) and GFP-SCAP in green. After isolation, cholesterol + acyl-CoA was added to stimulate cholesterol ester (CE) synthesis.

On the left panel, the formation of ER domains is observed by the increasing heterogeneity of GFP-SCAP around the GERVs. On the middle panel, ACAT is inhibited using the drug Sandoz 58-035 2 hours prior to GERVs formation and kept during the whole experiment in the hypotonic media, blocking the synthesis of CE. In that case, the signal of GFP-SCAP on GERVs stays homogeneous throughout the experiment. On the right panel, DGATs (diacylglycerol O-acyltransferases) which synthetise triglycerides are inhibited and the inhibition did not prevent domain formation.

All together these data highlight the importance of cholesterol ester in the formation of ER domains and demonstrate the strengths of GERVs as a tool to probe these domains.

The examples given above are only preferred embodiments of the invention and are not intended to limit the scope of the present invention. Modifications, replacements by equivalents and improvements made by persons skilled in the art without departing from the spirit of the present invention must come within the framework of the present invention defined by the appended claims.

## Claims

1. Method for screening the activity of a molecule involved in the regulation of organelle domains, said method comprising the following steps :
a) providing giant organelle vesicles, GOVs ;
b) supplementing the GOVs with at least a molecule to be tested;
c) screening the molecule based on its capacity to induce a modification of the organelle domains in the GOVs after the supplementation,
wherein the giant organelle vesicles are chosen from the group consisting of the endoplasmic reticulum, mitochondria, lysosome, nucleus, Golgi apparatus, vacuole, chloroplast, autophagosome, autolysosomes, endosomes, plasma membranes, peroxisome, multivesicular bodies.

2. Method for screening the activity of a molecule according to claim 1, wherein the giant organelle vesicles are giant endoplasmic reticulum vesicules (GERVs), said method comprising the following steps :
a) providing giant endoplasmic reticulum vesicles (GERVs) ;
b) supplementing the GERVs with at least a molecule to be tested;
c) screening the molecule based on the modification of the ER domains in the GERVs after the supplementation.

3. Method for screening the activity of a molecule according to any of claims 1-2, wherein the molecules to be tested are chosen from the group consisting of molecules involved in protein regulation and lipid metabolism.

4. Method for screening the activity of a molecule according to any of claims 1-3, wherein the molecules to be tested are chosen from the group consisting of molecules involved in the metabolism of cholesterol, such as molecules involved in the reactions of esterification of cholesterol, hydrolysis of cholesterol ester back to free cholesterol and fatty acids, and their regulation, molecules involved in the metabolism of saturated lipids or molecules involved in the metabolism of sphingolipids.

5. Method for modulating organelle domain formation, said method comprising the following steps :
a) providing giant organelle vesicles, GOVs ;
b) supplementing the GOVs with at least a molecule identified in any one of the methods of claims 1-4 as having an effect on the domain formation, composition, and properties,
wherein said domains are to be used for studying cellular processes dependent on the localization and activation of specific proteins,
wherein the giant organelle vesicles are chosen from the group consisting of the endoplasmic reticulum, mitochondria, lysosome, nucleus, Golgi apparatus, vacuole, chloroplast, autophagosome, autolysosomes, endosomes, plasma membranes, peroxisome, multivesicular bodies.

6. Method for modulating organelle domain formation, said method comprising the following steps:
a) providing giant endoplasmic reticulum vesicles, GERVs, in particular ;
b) supplementing the GERVs with at least a molecule identified in any one of the methods of claims 1-4 as having an effect on the domain formation, composition, and properties,
wherein said domains are to be used for studying cellular processes dependent on the localization and activation of specific proteins.

7. Use of giant organelle vesicles for studying organelle domain formation, wherein the giant organelle vesicles are chosen from the group consisting of the endoplasmic reticulum, mitochondria, lysosome, nucleus, Golgi apparatus, vacuole, chloroplast, autophagosome, autolysosomes, endosomes, plasma membranes, peroxisome, multivesicular bodies.

8. Use of giant organelle vesicles in a method for screening the activity of a molecule or combination of molecules involved in the modulation of organelle domains according to any of claims 1-4.

9. Use according to claim 8, wherein the giant organelle vesicles are giant endoplasmic reticulum vesicles (GERVs) and wherein the molecule or combination of molecules is involved in modulating ER domains.

10. Use according to any of claims 8-9, wherein the molecule or combination of molecules is involved in the regulation of the INSIG/SCAP/SREBP complex in the endoplasmic reticulum.

11. Use according to any of claims 9-10, wherein the molecule is chosen from the group comprising a combination of cholesterol and acyl-CoA, an inhibitor of ACAT, such as Sandoz 58-035, an inhibitor of NCEH1, an inhibitor of SPT, an inhibitor of DGAT, lipid synthesis enzyme inhibitors.

12. Screening platform for identifying domain-modulating molecules in organelles, wherein said platform uses the giant organelle vesicles technology and wherein the giant organelle vesicles are chosen from the group consisting of the endoplasmic reticulum, mitochondria, lysosome, nucleus, Golgi apparatus, vacuole, chloroplast, the autophagosome, autolysosomes, endosomes, plasma membranes, peroxisome, multivesicular bodies.

13. Molecule or combination of molecules identified by the screening method of any of claims 1-4, for use in the prevention and/or treatment of diseases, disorders or deficiencies related to the protein regulation and/or lipid metabolism.

14. Molecule or combination of molecules according to claim 13 for use in the prevention and/or treatment of diseases, disorders or deficiencies related to cholesterol metabolism.
